# EUROPÄISCHE PATENTSCHRIFT

(11) **EP 0 523 767 B1**
(45) Veröffentlichungstag und Bekanntmachung des Hinweises auf die Patenterteilung: **13.12.1995**
(21) Anmeldenummer: 92201804.9
(22) Anmeldetag: 19.06.1992
(51) Int. Cl.: C07C 67/03, C07C 69/24, C07C 69/52

(54) **Verfahren zum Erzeugen von Fettsäure-Methylester oder Fettsäure-Äthylester und Glycerin durch Umesterung von Ölen oder Fetten**
Process for the production of methyle or ethyle esters from fatty acids and glycerine by transesterification of oils or greases
Procédé de fabrication d'esters de méthyle ou d'éthyle d'acides gras et de glycérine par transestérification d'huiles ou de graisses

(30) Priorität: 19.07.1991 DE 4123928
(43) Veröffentlichungstag der Anmeldung: 20.01.1993
(73) Patentinhaber: METALLGESELLSCHAFT Aktiengesellschaft, 60015 Frankfurt (DE)
(72) Erfinder: Voeste, Theodor, W-6000 Frankfurt am Main (DE); Buchold, Henning, Dr., W-6450 Hanau am Main (DE); Aalrust, Erik, W-6104 Seeheim-Jugenheim (DE); Saft, Helmut, W-6361 Niddatal 1 (DE); Schmidt, Hans-Joachim, Dr., W-6000 Frankfurt am Main (DE); Mallok, Gerd, W-6450 Hanau am Main (DE); Ludwig, Ernst, W-6370 Oberursel (DE)

(56) Entgegenhaltungen:
- WO-A-91/05034
- DE-A- 3 020 612
- DE-A- 3 107 318

## Beschreibung

Die Erfindung betrifft ein Verfahren zur Umsetzung eines Öls oder Fettes mit Methanol oder Äthanol und einem alkalischen Katalysator in flüssiger Phase zu den Produkten Fettsäure-Methylester oder Fettsäure-Äthylester und Glycerin, die getrennt gewonnen werden, dabei arbeitet man mit mindestens zwei Umsetzungsstufen, wobei jede Umsetzungsstufe einen Mischreaktor und einen Abscheider zur Abtrennung einer leichten, esterreichen Phase und einer schweren, glycerinreichen Phase aufweist, man führt dem Mischreaktor der ersten Umsetzungsstufe Öl oder Fett sowie Methanol oder Äthanol und Katalysator zu und vermischt diese Substanzen intensiv und man führt dem Mischreaktor der zweiten und jeder weiteren Umsetzungsstufe zum intensiven Vermischen Methanol oder Äthanol und Katalysator sowie die in der vorausgehenden Umsetzungsstufe gewonnene leichte, esterreiche Phase zu. Als Ausgangsmaterial wird zumeist ein pflanzliches Öl oder Fett verwendet werden, doch ist auch ein fließfähiges tierisches Öl oder Fett nicht ausgeschlossen.

Ein Verfahren der eingangs genannten Art ist aus WO-A-91 05 034 bekannt.

Der Erfindung liegt die Aufgabe zugrunde, das Verfahren auf einfache und kostengünstige Weise durchzuführen, gleichzeitig sollen hohe Umsätze erreicht werden.

Erfindungsgemäß geschieht dies dadurch, daß man die im Abscheider der zweiten bis letzten Umsetzungsstufe abgetrennte schwere Phase mindestens teilweise zurück zum Mischreaktor der ersten Umsetzungsstufe führt und daß man aus der in der letzten Umsetzungsstufe abgetrennten leichten, esterreichen Phase Fettsäure-Methylester oder Fettsäure-Äthylester durch mindestens eine weitere Separationsbehandlung gewinnt.

Die Menge des insgesamt den verschiedenen Umsetzungsstufen zugeführten Methanols oder Äthanols liegt im Bereich vom 1- bis 3-fachen der stöchiometrisch notwendigen Menge. Die zu verwendenden alkalischen Katalysatoren sind an sich bekannt, es kann sich dabei z.B. um NaOH, KOH oder um Natriummethylat handeln. Man verwendet den Katalysator im allgemeinen in einer Konzentration von 0,1 bis 1 Gew.%, bezogen auf umzusetzendes Öl oder Fett.

Das Verfahren wird in den meisten Fällen mit zwei oder drei Umsetzungsstufen durchgeführt. Wenn man nur mit begrenztem apparativem Aufwand arbeiten will, empfehlen sich zwei Umsetzungsstufen.

Wenn man mit zwei Umsetzungsstufen arbeitet, ist es zweckmäßig, dem Mischreaktor der ersten Umsetzungsstufe 20 bis 60 % und vorzugsweise 35 bis 45 % des gesamten Methanols oder Äthanols sowie 20 bis 60 % und vorzugsweise 30 bis 50 % des gesamten Katalysators zuzuführen. Den Rest des Methanols oder Äthanols und des Katalysators gibt man in den Mischreaktor der zweiten Umsetzungsstufe. Bei der hier gemeinten Gesamtmenge an Methanol oder Äthanol und Katalysator werden die kleinen Mengen nicht mitgerechnet, die durch Rückführen der schweren Phase aus dem Abscheider der zweiten Umsetzungsstufe in den Mischreaktor der ersten Umsetzungsstufe gelangen.

Aus dem Abscheider der ersten Umsetzungsstufe zieht man eine schwere, glycerinreiche Phase ab und unterzieht sie vorzugsweise einer weiteren Trennbehandlung. Hierbei kann man ein weitgehend wasserfreies Methanol oder Äthanol zurückgewinnen, das man in die Umesterung zurückführt. Aus dem Abscheider der letzten Umsetzungsstufe erhält man eine leichte, esterreiche Phase, aus welcher man durch mindestens eine weitere Separationsbehandlung Fettsäure-Methylester oder Fettsäure-Äthylester gewinnt. Diese weitere Separationsbehandlung kann z.B. darin bestehen, die Phase mit Wasser zu vermischen und das Gemisch anschließend zu zentrifugieren. Aus der Zentrifuge erhält man den Ester mit einem Wassergehalt von höchstens 2 Gew.% und daneben eine wäßrige, alkoholhaltige Phase.

Um die Trennung der Phasen in mindestens einem Abscheider zu erleichtern, kann es sich empfehlen, das Flüssigkeitsgemisch vor dem Abscheider so zu kühlen, daß sich die Temperatur um 5 bis 50°C und vorzugsweise 10 bis 30°C verringert.

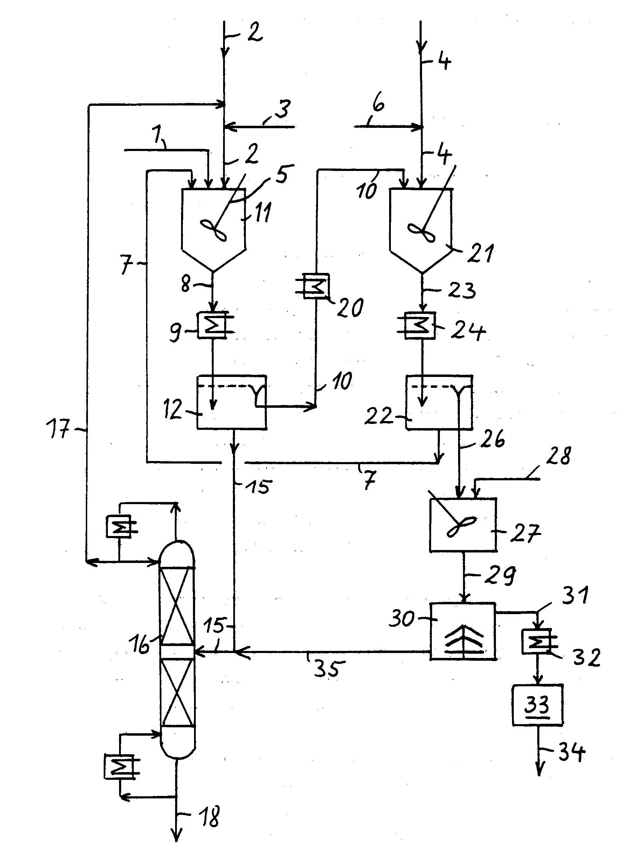

Ausgestaltungsmöglichkeiten des Verfahrens mit zwei Umsetzungsstufen werden mit Hilfe der Zeichnung erläutert. Hierbei wird als Alkohol Methanol eingesetzt, doch kann das Verfahren in gleicher Weise auch mit Äthanol zur Erzeugung von Äthylester durchgeführt werden.

Die erste Umsetzungsstufe weist den Mischreaktor (11) und den Schwerkraftabscheider (12) auf, zur zweiten Umsetzungsstufe gehören der Mischreaktor (21) und der Schwerkraftabscheider (22). Das zu behandelnde Öl oder Fett fließt durch die Leitung (1) in den Mischreaktor (11). Dem Reaktor (11) führt man ferner durch die Leitung (2) weitgehend wasserfreies Methanol und Katalysator sowie durch die Leitung (7) schwere, glycerinhaltige Phase aus der zweiten Umsetzungsstufe zu. Der Katalysator wird durch die Leitung (3) herangeführt und mit dem Methanol gemischt. Im Reaktor (11) erfolgt eine intensive Durchmischung mit Hilfe von mindestens einer Rühreinrichtung (5). Der Mischreaktor (11) kann in bekannter Weise mehrere hintereinandergeschaltete Mischkammern aufweisen, was in der Zeichnung nicht dargestellt ist.

Es empfiehlt sich, die in der Leitung (2) in den Reaktor (11) eintretende Menge an Methanol und Katalysator so zu bemessen, daß es sich um 20 bis 60 % des gesamten dem Umsetzungsverfahren beider Stufen zugeführten Methanols und um 20 bis 60 % des gesamten Katalysators handelt. Vorzugsweise gibt man in den Reaktor (11) 35 bis 45 % des gesamten Methanols und 30 bis 50 % des gesamten Katalysators.

Die Temperaturen im Mischreaktor (11) liegen üblicherweise im Bereich von 50 bis 90°C und man arbeitet zumeist bei Atmosphärendruck. Es kann zweckmäßig sein, das in der Leitung (8) aus dem Reaktor (11) kommende Gemisch zunächst etwas zu kühlen, wofür der Kühler (9) vorgesehen ist, um die Trennung im Abscheider (12) zu erleichtern. Bei Verwendung des Kühlers (9) senkt man die Temperatur an dieser Stelle um 5 bis 50°C und vorzugsweise 10 bis 30°C. Es ist aber auch möglich, ohne den Kühler (9) zu arbeiten.

Als Schwerkraft-Abscheider verwendet man bevorzugt Faserbettabscheider, die an sich bekannt und z.B. in "Filtration & Separation" (Sept./Oct. 1990), S. 360-363, beschrieben sind.

Im Abscheider (12) zieht man eine leichte, methylesterreiche Phase ab, die man durch die Leitung (10) zum Mischreaktor (21) der zweiten Umsetzungsstufe führt. Die in der Leitung (15) abgezogene schwere, glycerinreiche Phase wird zur Rektifikation einer Kolonne (16) aufgegeben. In der an sich bekannten Rektifikationskolonne gewinnt man über Kopf weitgehend wasserfreies Methanol, das man in der Leitung (17) abzieht und dem Methanol der Leitung (2) zugibt. Aus dem Sumpf der Kolonne (16) zieht man durch die Leitung (18) rohes Glycerin ab, das einer nicht dargestellten Reinigung zugeführt wird.

Der zweiten Umsetzungsstufe führt man ein Gemisch aus Methanol und Katalysator durch die Leitung (4) zu, wobei der Katalysator in der Leitung (6) herangeführt wird. Vor Eintritt in den Mischreaktor (21) kann die Temperatur der methylesterreichen Phase der Leitung (10) in einem Kühler (20) abgesenkt werden, doch ist dies nicht unbedingt erforderlich. Falls man sich zum Verwenden des Kühlers (20) entschließt, erfolgt hier eine Kühlung um vorzugsweise 10 bis 40°C. Im Mischreaktor (21), in welchem wiederum eine intensive Vermischung erfolgt, ist die Temperatur etwa die gleiche wie im Mischreaktor (11) oder sie liegt um etwa 5 bis 50°C und vorzugsweise 10 bis 30°C niedriger als im Mischreaktor (11). Das in der Leitung (23) ablaufende Gemisch kann vor dem Abscheider (22) wiederum gekühlt werden, wofür ein Kühler (24) vorgesehen ist. Hier wie bereits im Kühler (9) kann die Temperatur um 5 bis 50°C oder vorzugsweise 10 bis 30°C abgesenkt werden.

Die schwere, glycerinreiche Phase, die im Abscheider (22) anfällt, wird durch die Leitung (7) zum Mischreaktor (11) geführt. Die leichte, methylesterreiche Phase gelangt durch die Leitung (26) zu einem Extraktor (27), dem man durch die Leitung (28) Wasser zuführt. Das Wasser nimmt die wasserlöslichen Substanzen, vor allem Methanol und Glycerin, auf und zerstört den Katalysator. Das im Extraktor (27) erzeugte Gemisch führt man durch die Leitung (29) zu einer Zentrifuge (30), aus der man durch die Leitung (31) Fettsäure-Methylester mit einem Wassergehalt von höchstens 2 Gew.% und vorzugsweise höchstens 0,5 Gew.% abzieht. Diese Reinheit kann für bestimmte Anwendungsfälle bereits ausreichen. Will man den Wassergehalt weiter senken, so erhitzt man das Produkt im Wärmeaustauscher (32) und unterwirft es einer Trocknung (33), z.B. einer Vakuumtrocknung, aus der man dann durch die Leitung (34) ein weitgehend getrocknetes Produkt abführt.

Die in der Zentrifuge (30) gebildete wäßrige Phase, die Methanol und Glycerin enthält und praktisch frei von Methylester ist, wird in der Leitung (35) der glycerinreichen Phase der Leitung (15) zugemischt, die man der Kolonne (16) aufgibt.

### Beispiel

In einer der Zeichnung entsprechenden Verfahrensführung ohne den Kühler (9) werden pro Stunde 1000 kg Rapsöl umgesetzt, die man in der Leitung (1) heranführt. Das Rapsöl war zuvor durch Entschleimung, Bleichung und Entsäuerung vorbehandelt worden. Die Mischreaktoren (11) und (21) weisen jeweils drei hintereinandergeschaltete Mischkammern auf und die Abscheider (12) und (22) sind als Faserbettabscheider ausgebildet.

In den Mischreaktor (11) werden pro Stunde 74 kg Methanol und als Katalysator 2,4 kg Natriummethylat eingeleitet, die Durchmischung erfolgt bei 75°C und Atmosphärendruck. Die leichte, methylesterreiche Phase der Leitung (10) wird im Kühler (20) auf 60°C abgekühlt, bevor sie in den Mischreaktor (21) eintritt. Diesem Mischreaktor führt man pro Stunde 118 kg Methanol und 3,6 kg Natriummethylat zu. Im Reaktor (21) arbeitet man bei 60°C und Atmosphärendruck.

Im Kühler (24) wird das Gemisch auf 35°C abgekühlt und man zieht in der Leitung (26) einen rohen Fettsäure-Methylester ab, welcher 99,2 % der im öl enthaltenen Fettsäuren als Methylester enthält.

Im Extraktor (27) wird der rohe Methylester mit 54 kg pro Stunde Waschwasser versetzt und so von gelöstem Methanol und Glycerin befreit. In der Zentrifuge (30) wird der weitgehend methanol- und glycerinfreie Methylester vom Waschwasser getrennt, und um den Wassergehalt im Fettsäure-Methylester weiter zu reduzieren, erfolgt eine Vakuumtrocknung (33) nach vorhergehender Aufwärmung im Wärmeaustauscher (32). In der Leitung (34) fallen pro Stunde 1001 kg Fettsäure-Methylester in der Qualität von Dieselöl-Ersatzkraftstoff an. In der Rektifikationskolonne (16) arbeitet man bei Atmosphärendruck und 110°C Sumpftemperatur, man zieht in der Leitung (18) pro Stunde 169 kg methanolfreie wäßrige Glycerinphase und in der Leitung (17) 81,3 kg wasserfreies Methanol ab.

## Patentansprüche

1. Verfahren zur Umsetzung eines Öls oder Fettes mit Methanol oder Äthanol und einem alkalischen Katalysator in flüssiger Phase zu den Produkten Fettsäure-Methylester oder Fettsaure-Äthylester und Glycerin, die getrennt gewonnen werden, dabei arbeitet man mit mindestens zwei Umsetzungsstufen, wobei jede Umsetzungsstufe einen Mischreaktor und einen Abscheider zur Abtrennung einer leichten, esterreichen Phase und einer schweren, glycerinreichen Phase aufweist, man führt dem Mischreaktor der ersten Umsetzungsstufe Öl oder Fett sowie Methanol oder Äthanol und Katalysator zu und vermischt diese Substanzen intensiv und man führt dem Mischreaktor der zweiten und jeder weiteren Umsetzungsstufe zum intensiven Vermischen Methanol oder Äthanol und Katalysator sowie die in der vorausgehenden Umsetzungsstufe gewonnene leichte, esterreiche Phase zu, dadurch gekennzeichnet, daß man die im Abscheider der zweiten bis letzten Umsetzungsstufe abgetrennte schwere Phase mindestens teilweise zurück zum Mischreaktor der ersten Umsetzungsstufe führt und daß man aus der in der letzten Umsetzungsstufe abgetrennten leichten, esterreichen Phase Fettsäure-Methylester oder Fettsäure-Äthylester durch mindestens eine weitere Separationsbehandlung gewinnt.

2. Verfahren nach Anspruch 1, dadurch gekennzeichnet, daß man mit zwei Umsetzungsstufen arbeitet und dem Mischreaktor der ersten Umsetzungsstufe 20 bis 60 % des gesamten Methanols oder Äthanols und 20 bis 60 % des gesamten Katalysators zuführt und den Rest des Methanols oder Äthanols und des Katalysators in den Mischreaktor der zweiten Umsetzungsstufe leitet.

3. Verfahren nach Anspruch 2, dadurch gekennzeichnet, daß man dem Mischreaktor der ersten Umsetzungsstufe 35 bis 45 % des gesamten Methanols oder Äthanols und 30 bis 50 % des gesamten Katalysators zuführt.

4. Verfahren nach Anspruch 1 oder einem der folgenden, dadurch gekennzeichnet, daß man die aus dem Abscheider der ersten Umsetzungsstufe abgezogene schwere, glycerinreiche Phase einer Trennbehandlung unterwirft und dabei Methanol oder Äthanol vom Glycerin abtrennt.

5. Verfahren nach Anspruch 1, dadurch gekennzeichnet, daß die weitere Separationsbehandlung, welcher man die leichte, esterreiche Phase aus der letzten Umsetzungsstufe aufgibt, aus einer Extraktion mit Wasser und anschließender Zentrifugierung besteht, wobei man als getrennte Produkte Fettsäure-Methylester oder Fettsäure-Äthylester mit einem Wassergehalt von höchstens 2 Gew.% und eine wasserreiche Phase erhält.

6. Verfahren nach Anspruch 5, dadurch gekennzeichnet, daß man die wasserreiche Phase zusammen mit der schweren, glycerinreichen Phase aus dem Abscheider der ersten Umsetzungsstufe weiterbehandelt.

7. Verfahren nach Anspruch 1 oder einem der folgenden, dadurch gekennzeichnet, daß in mindestens einer Umsetzungsstufe zwischen dem Mischreaktor und dem Abscheider eine Kühlung mit Verringerung der Temperatur um 5 bis 50°C und vorzugsweise 10 bis 30°C erfolgt.

## Claims

1. A method for reacting an oil or fat with methanol or ethanol and an alkaline catalyst in liquid phase to yield the products fatty acid methyl enter or fatty acid ethyl ester and glycerol, which are obtained separately, wherein one operates with at least two reaction stages, each reaction stage having a mixing-reactor and a separator for separating off a light, ester-rich phase and a heavy, glycerol-rich phase, oil or fat and methanol or ethanol and catalyst are supplied to the mixing-reactor of the first reaction stage and these substances are thoroughly mixed, and methanol or ethanol and catalyst and the light, ester-rich phase obtained in the preceding reaction stage are fed to the mixing-reactor of the second and each additional reaction stage for intensive mixing, characterized in that the heavy phase separated off in the separator of the second to final reaction stage is at least partly returned to the mixing-reactor of the first reaction stage and that fatty acid methyl ester or fatty acid ethyl ester is recovered from the light, ester-rich phase separated off in the final reaction stage by at least one additional separation treatment.

2. A method according to Claim 1, characterised in that one operates using two reaction stages and 20 to 60% of the total methanol or ethanol and 20 to 60% of the total catalyst are fed to the mixing-reactor of the first reaction stage and the rest of the methanol or ethanol and of the catalyst is passed into the mixing-reactor of the second reaction stage.

3. A method according to Claim 2, characterised in that 35 to 45% of the total methanol or ethanol and 30 to 50% of the total catalyst are fed to the mixing-reactor of the first reaction stage.

4. A method according to Claim 1 or one of the following claims, characterised in that the heavy, glycerol-rich phase withdrawn from the separator of the first reaction stage is subjected to a separation treatment wherein methanol or ethanol is separated off from the glycerol.

5. A method according to Claim 1, characterised in that the further separation treatment to which the light, ester-rich phase from the final reaction stage is fed consists of extraction with water and subsequent centrifugation, with fatty acid methyl ester or fatty acid ethyl ester having a water content of at most 2% by weight and a water-rich phase being obtained as separated products.

6. A method according to Claim 5, characterised in that the water-rich phase is treated further together with the heavy, glycerol-rich phase from the separator of the first reaction stage.

7. A method according to Claim 1 or one of the following claims, characterised in that cooling with a reduction of the temperature by 5 to 50°C and preferably 10 to 30°C is effected in at least one reaction stage between the mixing-reactor and the separator.

## Revendications

1. Procédé pour la mise en réaction d'une huile ou d'une graisse avec du méthanol ou de l'éthanol et un catalyseur alcalin en phase liquide pour obtenir les produits d'esters méthyliques d'acides gras ou d'esters éthyliques d'acides gras et de glycérine que l'on obtient séparément, en travaillant avec au moins deux étapes réactionnelles, dans lequel chaque étape réactionnelle présente un réacteur de mélange et un séparateur pour la séparation d'une phase légère riche en esters et d'une phase lourde riche en glycérine, on achemine dans le réacteur de mélange de la première étape réactionnelle l'huile ou la graisse, ainsi que le méthanol ou l'éthanol et le catalyseur et on mélange intimement ces substances, et on guide dans le réacteur de mélange de la deuxième étape réactionnelle et de chaque étape réactionnelle ultérieure, à des fins de mélange intime, le méthanol ou l'éthanol et le catalyseur, ainsi que la phase légère riche en esters obtenue à l'étape réactionnelle antérieure, caractérisé en ce qu'on recycle au moins partiellement dans le réacteur de mélange de la première étape réactionnelle la phase lourde séparée dans le séparateur de la deuxième à la dernière étape réactionnelles et en ce qu'on récupère, à l'aide d'au moins un traitement de séparation ultérieur, des esters méthyliques d'acides gras ou des esters éthyliques d'acides gras à partir de la phase légère riche en esters séparée dans la dernière étape réactionnelle.

2. Procédé selon la revendication 1, caractérisé en ce qu'on travaille avec deux étapes réactionnelles et en ce qu'on achemine dans le réacteur de mélange de la première étape réactionnelle de 20 à 60 % du méthanol ou de l'éthanol total et de 20 à 60 % du catalyseur total et on guide le reste du méthanol ou de l'éthanol et du catalyseur dans le réacteur de mélange de la deuxième étape réactionnelle.

3. Procédé selon la revendication 2, caractérisé en ce qu'on achemine dans le réacteur de mélange de la première étape réactionnelle de 35 à 45 % du méthanol ou de l'éthanol total et de 30 à 50 % du catalyseur total.

4. Procédé selon la revendication 1, ou selon l'une quelconque des revendications suivantes, caractérisé en ce qu'on soumet à un traitement de séparation la phase lourde riche en glycérine extraite du séparateur de la première étape réactionnelle et on sépare en l'occurrence le méthanol ou l'éthanol de la glycérine.

5. Procédé selon la revendication 1, caractérisé en ce que le traitement de séparation ultérieur que l'on impose à la phase légère riche en esters provenant de la dernière étape réactionnelle, est constitué par une extraction dans de l'eau et par une centrifugation subséquente, dans lequel on obtient, comme produits séparés, des esters méthyliques d'acides gras ou des esters éthyliques d'acides gras présentant une teneur en eau maximale de 2 % en poids et une phase riche en eau.

6. Procédé selon la revendication 5, caractérisé en ce qu'on soumet à un traitement ultérieur la phase riche en eau, conjointement avec la phase lourde riche en glycérine provenant du séparateur de la première étape réactionnelle.

7. Procédé selon la revendication 1, ou selon l'une quelconque des revendications suivantes, caractérisé en ce que, dans au moins une étape réactionnelle entre le réacteur de mélange et le séparateur, a lieu un refroidissement en diminuant la température de 5 à 50°C, de préférence de 10 à 30°C.
